# EUROPÄISCHE PATENTSCHRIFT

(11) **EP 2 845 559 B1**
(45) Veröffentlichungstag und Bekanntmachung des Hinweises auf die Patenterteilung: **13.06.2018**
(21) Anmeldenummer: 14183900.1
(22) Anmeldetag: 08.09.2014
(51) Int. Cl.: A61C 1/00, A61C 1/07, A61C 1/08, A61C 17/20, A61G 15/14, A61B 5/22, A61B 5/00

(54) **Steuerung eines Geräts zur Behandlung eines Patienten**
Control of a device for treating a patient
Commande d'un appareil destiné au traitement d'un patient

(30) Priorität: 06.09.2013 DE 102013217891
(43) Veröffentlichungstag der Anmeldung: 11.03.2015
(73) Patentinhaber: Sirona Dental Systems GmbH, 64625 Bensheim (DE)
(72) Erfinder: Sutter, Ralf, 69469 Weinheim (DE); Oehme, Bernd, 55128 Mainz (DE)
(74) Vertreter: Sommer, Peter

(56) Entgegenhaltungen:
- EP-A1- 2 359 747
- WO-A1-2008/000446
- GB-A- 727 515
- US-A1- 2011 066 078

## Beschreibung

### Gebiet der Erfindung

Die Erfindung betrifft ein dentales Gerät zur Behandlung eines Patienten, z. B. ein Ultraschall-Behandlungsgerät oder ein AirScaler, mit einem Mittel zum Steuern des Geräts. Solche Geräte kommen vor allem im Zahnarztbereich zum Einsatz, beispielsweise bei der Behandlung von Parodontitis oder bei der Zahnsteinentfernung.
Bei der Behandlung von Parodontitis werden zunächst der Erkrankungszustand des Zahnfleischs und des Zahnhalteapparats festgestellt und dann Plaque, Zahnstein sowie die pathogene Bakterienflora (Biofilm) beseitigt. Dabei werden im Rahmen einer sog. professionellen Zahnreinigung zunächst alle supragingival (oberhalb des Zahnfleischrands) gelegenen harten und weichen Beläge entfernt. Im Anschluss beginnt bei Bedarf die eigentliche Parodontitistherapie, bei der die subgingival (unterhalb des Zahnfleischrands) liegenden harten und weichen Beläge entfernt werden (geschlossenes parodontales Debridement). Dabei werden die Zahn- und Wurzeloberflächen mit Küretten (speziell geformten Handinstrumenten), mit schall- und ultraschallbetriebenen Geräten gereinigt und geglättet , damit der Zahnhalteapparat ausheilen kann. Diese Maßnahmen müssen ggf. im zeitlichen Abstand an einzelnen Stellen wiederholt werden.
Vorteil: Patienten, die frei von Angst vor dem Schmerz bei der Behandlung sind, gehen regelmäßiger zur Behandlung, was insbesondere bei chronischen Erkrankungen (Parodontitis) von besonderer Bedeutung für die Beherrschung der Krankheit ist.

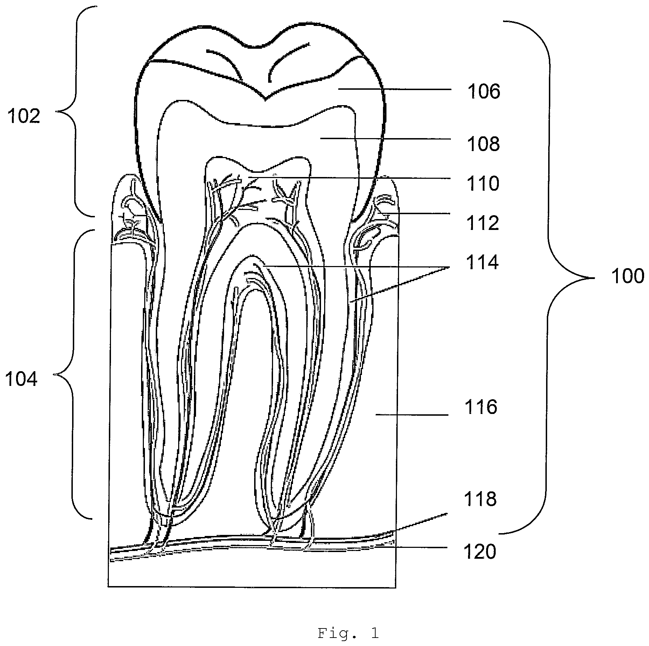

Die Behandlung erfolgt in der heutigen Zeit typischerweise mit schall- oder ultraschallbetriebenen Geräten (piezoelektrisch oder magnetostriktiv) und den dazugehörigen Applikationsspitzen. Der Schmerz entsteht dadurch, dass die schwingende Applikationsspitze zum Zwecke der Biofilm- oder Konkrement-Entfernung mit der Zahnoberfläche in Kontakt treten muss, also durch die mechanische Interaktion mit dem Gewebe. Insbesondere bei fortgeschrittener Erkrankung - hier ist eine regelmäßige Behandlung zwingend notwendig - treten bei tiefen Zahnfleischtaschen häufig Schmerzen bei der Behandlung auf, insbesondere am Zahnhals. Der Schmerz wird dann größer, wenn die Zementschicht, die das Dentin überlagert, durch Putzdefekte oder häufige Behandlungen geschädigt oder entfernt wurde. Freiliegende Dentintubuli und Druckänderungen in den lateralen Dentintubuli führen u.a. zum Schmerzempfinden (vgl. hierzu Fig. 1 mit zugehöriger Beschreibung weiter unten). Zusätzlich kann die Interaktion der Applikationsspitze mit Weichgewebe zu Schmerzinduktionen führen. In beiden Fällen ist das Schmerzempfinden direkt von der Bearbeitungsleistung des Behandlungsgerätes abhängig. Schmerzen können darüber hinaus auch durch falsches Handling der Applikationsspitze entstehen.

### Stand der Technik

Treten bei den heutigen Behandlungen Schmerzen auf, kann dies der Behandler entweder an der körperlichen Reaktion des Patienten erkennen, oder es wurden im Vorfeld beispielsweise Handzeichen vereinbart, die das Auftreten von starken Schmerzen signalisieren sollen. In beiden Fällen findet keine direkte Einwirkung des Patienten auf die Bearbeitungsleistung des Behandlungsgerätes statt.

Erkennt der Zahnarzt heute, dass der Patient Schmerzen bei der Behandlung erleidet, ergeben sich folgende Optionen:
a) Einlegen einer Behandlungspause, allerdings mit dem Nachteil, dass sich dadurch die Behandlungszeit verlängert, bzw. die empfindliche Stelle noch einmal bearbeitet werden muss.
b) Reduktion der Leistung des Scalers (siehe Glossar weiter unten) - diese kann entweder kurzzeitig erfolgen, wobei die Leistung anschließend wieder erhöht wird, oder aber für die gesamte Dauer der Behandlung. Im Falle einer kurzzeitigen Reduktion tritt der Schmerz im weiteren Verlauf der Behandlung an einem anderen Zahn ggf. wieder auf, was für alle Beteiligten unbefriedigend ist. Im Falle einer dauerhaften Reduktion der Leistung des Scalers verlängert sich die Dauer der Behandlung, was ebenso nachteilig ist.
c) Anästhesie.

Aus der GB 727,515 ist ein Dentalgerät bekannt, bei dem der Betriebsstrom eines Motors über einen vom Patienten beeinflussbaren Oszillator verändert werden kann, um dem Schmerzempfinden des Patienten zu entsprechen. Dabei kann in einer Ausführung der Motor sofort vollständig abgestellt werden oder in einer anderen Ausführung die Geschwindigkeit des Motors vom Patienten verändert werden, ohne die Arbeit des Zahnarztes zu unterbrechen.

Aus der US 2011/0066078 A1 ist bekannt, die Schmerzen eines einer Behandlung ausgesetzten Patienten mittels eines handgehaltenen Kraftsensors zu erfassen und ein Signal bereitzustellen, das den verschiedenen Schmerzzuständen des Patienten entspricht.

### Aufgabe

Aufgabe der Erfindung ist es, ein Mittel anzugeben, das es einem Arzt ermöglicht, eine Behandlung mit einem Behandlungsgerät in effektiver Weise an Schmerzen des Patienten anzupassen, und dabei die Nachteile des Standes der Technik zu vermeiden.

### Lösung

Diese Aufgabe wird durch die Erfindung mit den Merkmalen des unabhängigen Anspruchs gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt dieser Beschreibung gemacht. Vorgeschlagen wird ein dentales Gerät zur Behandlung eines Patienten, mit einem Mittel zum Steuern des Geräts, wobei die Behandlung mit dem Gerät Schmerzen hervorrufen kann, und wobei eine Veränderung einer Arbeitsweise des Geräts geeignet ist, die Schmerzen zu verringern. Das Mittel umfasst eine Kraftmesseinrichtung, welche durch den Patienten betätigbar ist, und eine Komponente, die abhängig von der gemessenen Kraft eine Veränderung der Arbeitsweise des Geräts hervorzurufen geeignet ist.
Die Komponente ist derart ausgebildet, dass sie die Arbeitsweise des Geräts graduell verändern kann. Dies kann entweder stetig oder in Stufen geschehen und ermöglicht eine wesentlich individuellere Anpassung der Behandlung als ein System, welches lediglich die Zustände "an" und "aus" kennt. Weiterhin sind Mittel zum Begrenzen des Umfangs, in dem die Komponente abhängig von der gemessenen Kraft die Arbeitsweise des Geräts verändert, vorgesehen. Dadurch kann der behandelnde (Zahn-)Arzt die Grenzen vorgeben, in denen sich die Leistung des Behandlungsgeräts durch den Patienten beeinflussen lässt.

Der Patient ist dabei i. d. R. ein Mensch und muss bei der Behandlung bei Bewusstsein sein. Typischerweise kommt das Mittel bei einer Parodontitis-Behandlung zum Einsatz, allerdings sind auch andere Behandlungen, insbesondere im Dentalbereich, als Einsatzgebiete denkbar.

Als Kraftmesseinrichtung kommen dabei die unterschiedlichsten kraftgesteuerten Systeme in Betracht, so z. B. folgende:
a. Ein Ball oder Zylinder wird gedrückt und der Innendruck gemessen.
b. In einer flexiblen Leitung, z. B. einem Schlauch, durch die Luft strömt, wird der durch Handkraft beeinflusste Volumenstrom gemessen.
c. Ein Federelement wird zusammengedrückt, und die Krafterkennung erfolgt über eine Wegmessung (Federkennlinie).
d. Ein Element wird zusammengedrückt und nur der Weg gemessen. Die Kraft entspricht dabei dem Weg und kann in bekannter Weise daraus berechnet werden.
e. Der Winkel zwischen den beiden Schenkeln einer Klemme wird gemessen. Auch diese Umrechnung ist allgemein bekannt.
f. Messung mit Dehnungsmessstreifen.

Dabei wird vorzugsweise die Handkraft des Patienten gemessen. Alternativ ist z. B. auch eine Fußsteuerung möglich. Die Leistungsabgabe des Behandlungsgerätes kann mit dieser Steuerung durch den Patienten beeinflusst werden. Drückt der Patient gar nicht, dann läuft das Gerät mit der Leistung, die der behandelnde Arzt eingestellt hat. An Stelle der Leistungsabgabe kann beispielsweise auch die Frequenz z. B. eines Bohrers, die Amplitude, mit der ein schwingendes Element ausschlägt, oder auch der Abstand eines Geräts vom Patienten variiert werden.

Mit diesem Mittel wird in einfach zu handhabender Weise erreicht, dass der Schmerz, den der Patient bei der Behandlung erleidet, minimiert wird, da die Leistung des Geräts dann gesenkt wird, wenn der Patient Schmerzen empfindet, während das Gerät normal arbeitet, wenn der Patient keine Schmerzen empfindet. Auf dieses Weise wird die Behandlungszeit optimal an das Schmerzempfinden angepasst. Zugleich reagiert das Mittel unmittelbar und ohne Aufmerksamkeitsdefizite auf Schmerzen des Patienten, so dass der Arzt sich auf die Führung des Behandlungsgeräts konzentrieren kann und nicht gleichzeitig nach (möglicherweise verborgenen oder unterdrückten) Indikatoren für Schmerzen beim Patienten suchen muss.

Wird zusätzlich der zeitliche Verlauf der Betätigung der Kraftmesseinrichtung durch den Patienten aufgezeichnet, so ergeben sich weitere Steuerungsmöglichkeiten. Drückt ein Patient, z. B. aus Angst, die Kraftmesseinrichtung permanent, so könnte die Leistung des Behandlungsgeräts z. B. schleichend wieder erhöht werden.

Eine Festlegung einer unteren Grenze ist i. d. R. sinnvoll, um sicherzustellen, dass ein therapeutischer Nutzen (z. B. das Entfernen eines Belags) gewährleistet bleibt, auch wenn der Patient die Kraftmesseinrichtung betätigt. Auf diese Weise bleibt der Arzt Herr der Behandlung und der Patient kann die Behandlung nicht unterbrechen.

Alternativ dazu kann auch vorgesehen sein, dass der Patient eine Abschaltmöglichkeit hat, die die Behandlung unterbricht oder abbricht. Z. B. kann das Gerät ab Ausübung einer bestimmten Kraft durch den Patienten vollständig abgeschaltet werden.

Vorteilhafterweise ist außerdem ein Mittel zum Verhindern der Veränderung der Arbeitsweise des Geräts durch die Komponente vorgesehen. Dieses kann z. B. in Form eines Fußschalters für den Arzt oder einer Funkfernbedienung vorliegen, welche die Komponente abschalten kann. Dadurch kann der Arzt eingreifen, wenn beispielsweise ein Patient mit der Betätigung der Kraftmesseinrichtung nicht zurechtkommt. Oder der Arzt kann gegen den Willen des Patienten die Behandlung fortsetzen, etwa um noch den allerletzten kleinen Schritt zu Ende zu führen.

Besonders günstig ist es, wenn eine Anzeigeeinrichtung vorhanden ist, die anzeigen kann, ob die Komponente die Arbeitsweise des Geräts verändert, ob also der Patient die Behandlung beeinflusst. Dies kann beispielsweise optisch oder akustisch erfolgen. Die Anzeigeeinrichtung kann dabei am Gerät vorgesehen sein, oder auch an der Antriebseinheit bzw. Steuerung, oder ggf. einen vorhandenen Bildschirm o. Ä. nutzen.

Dies ist insbesondere deshalb von Vorteil, da der Behandler mit dieser Information die relative Position des Scalers bzw. der Applikationsspitze zum Zahn optimieren kann. Dem Fachmann ist bekannt, dass die richtige Handhabung von Scaler bzw. Applikationsspitze großen Einfluss auf mit der Behandlung verbundene Schmerzen hat.

Vorzugsweise wird das Mittel zum Steuern eines Geräts zur Behandlung eines Patienten in Zusammenhang mit einem Gerät zur Behandlung von Parodontitis eingesetzt, das auf Ultraschallbasis arbeitet (Einsatz an Stelle einer Kürette).

Das Mittel zum Steuern eines Geräts zur Behandlung eines Patienten ist Teil einer dentalen Behandlungseinheit (siehe Glossar weiter unten).

Weitere Einzelheiten und Merkmale ergeben sich aus der nachfolgenden Beschreibung eines bevorzugten Ausführungsbeispiels in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf das Ausführungsbeispiel beschränkt.

Ein Ausführungsbeispiel ist in den Figuren schematisch dargestellt. Im Einzelnen zeigen:
- Fig. 1: eine schematische Darstellung eines Zahns; und
- Fig. 2: eine schematische Darstellung der Mittel zum Steuern eines Geräts zur Behandlung eines Patienten.

Der Aufbau eines menschlichen Zahns 100 ist in Fig. 1 schematisch dargestellt. Der Zahn 100 besteht im Wesentlichen aus der Zahnkrone 102 und der Zahnwurzel 104 und ist aus mehreren Schichten aufgebaut. Bei einem gesunden Zahn sieht man nur den Zahnschmelz 106, der wie eine Glasur das innen liegende Dentin 108 bedeckt. Das Dentin 108 wiederum umschließt das Zahnmark (Pulpa) 110, in welchem sich auch Blutgefäße 118 und Nerven 120 befinden. Die Wurzel 104 wird von Zahnzement 114 und Wurzelhaut (fehlt in Fig. 1) umschlossen. Der komplette Wurzelbereich 104 wird normalerweise von Zahnfleisch 112 bzw. Kieferknochen 116 bedeckt.

Unter dem Zahnschmelz 106 liegt das Zahnbein bzw. Dentin 108. Es stellt die Hauptmasse des Zahnes 100 dar. Die Hartsubstanz des Dentins 108 besteht wie beim Zahnschmelz 106 aus Calcium und Phosphat, allerdings nur zu zwei Dritteln, der Rest ist Eiweiß und Wasser, weshalb Dentin weicher und anfälliger gegen Karies ist als der Zahnschmelz 106. Das Dentin 108 ist schmerzempfindlich. Hitze-, Kälte- und Berührungsreize führen zu Flüssigkeitsbewegungen in den Dentinkanälchen (Tubuli), die im Bereich des Übergangs von Wurzel 104 zu Krone 102 (Zahnhals) bis an die Oberfläche reichen können. Dies reizt die Tomes'schen Fasern, Zellfortsätze der Odontoblasten (dentinbildende Zellen). Die Odontoblasten stehen mit freien Nervenendungen 120 in Verbindung, die den Reiz als Schmerzempfindung ans Zentralnervensystem weiterleiten.

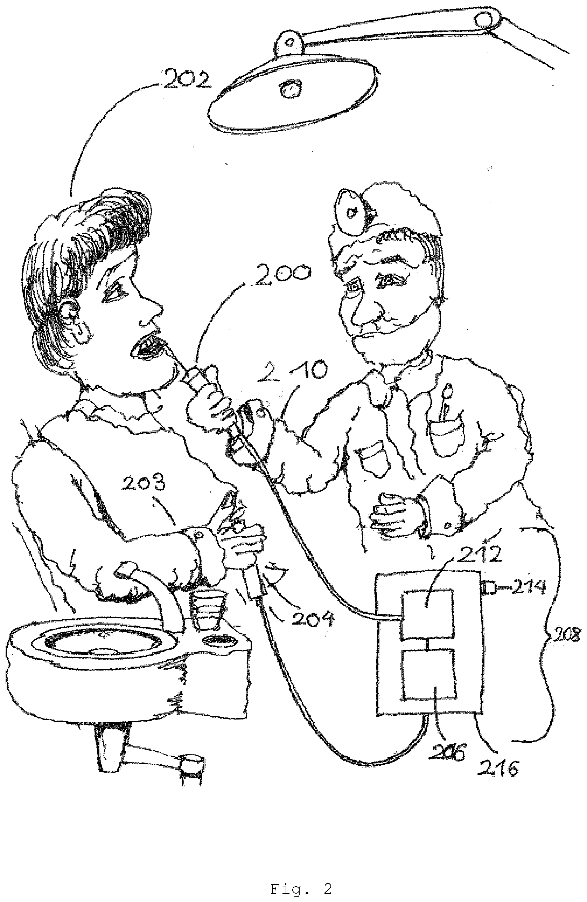

Im Folgenden wird auf Fig. 2 Bezug genommen. Ein ultraschallbetriebenes Behandlungsgerät 200 zur Behandlung von Parodontitis und zum Entfernen von Zahnstein weist eine Steuerung 208 auf, die die Ultraschallleistung (bzw. Antriebsleistung) 212 variieren kann. Ferner ist ein flexibler, hohler Ball oder Zylinder 204, z. B. aus Gummi, als Kraftmesseinrichtung vorgesehen. Der Patient 202 nimmt den Zylinder 204 in seine Hand 203 und drückt ihn zusammen, wenn er Schmerzen empfindet. Instinktiv wird der Druck umso stärker ausfallen, je höher das Schmerzempfinden des Patienten 202 ist. Der Druck in dem Zylinder 204 wird gemessen, und eine Komponente 206 der Steuerung 208 des Behandlungsgeräts 200 verringert die Ultraschalleistung 212 abhängig vom gemessenen Druck.

Dabei können die Grenzen, innerhalb derer die Ultraschalleistung 212 vom Patienten variiert werden kann, vom behandelnden Arzt 210 vorgegeben werden. Mittels der vorgegebenen unteren Grenze stellt der Arzt 210 sicher, dass die Behandlung stets einen therapeutischen Nutzen aufweist. Die obere Grenze ergibt sich aus der Leistung, die der Arzt 210 für die Behandlung gewählt hat. Zusätzlich verfügt die Steuerung 208 über einen unauffälligen Schalter 214, mit dem der behandelnde Arzt 210 bei Bedarf die Beeinflussung der Ultraschallleistung durch den Patienten 202 unterbinden kann. Das BehandlungsgeräL 200 und seine Steuerung 208 ist als Teil eines dentalen Carts 216 vorgesehen.

Es sind zahlreiche Abwandlungen und Weiterbildungen des beschriebenen Ausführungsbeispiels verwirklichbar. So kann die Steuerung 208 grundsätzlich für jede Art von Behandlungsgerät 200 eingesetzt werden, bei welchem die Beeinflussung eines Parameters des Geräts das Schmerzempfinden des Patienten 202 bei der Behandlung verringern kann. Die Kraftmessung kann mit einer beliebigen zusammendrückbaren Einrichtung 204 erfolgen. Ggf. kann es wünschenswert sein, bei Schmerzen das Gerät 200 komplett zu stoppen, anstatt einen Parameter in mehreren Stufen oder kontinuierlich zu verändern.

### Glossar

### AirScaler

Ein AirScaler ist ein mit Pressluft betriebenes Instrument, das eine Spitze zum Schwingen bringt. AirScaler werden über den Turbinenanschluss am Behandlungsstuhl mit Pressluft versorgt. Sie erreichen maximal 8000 Schwingungen pro Sekunde und arbeiten damit nicht im Ultraschallbereich, der durch Schwingungen oberhalb von ca. 20 kHz definiert ist. (Nach: "Einführung in die Zahnerhaltung"; von Elmar Hellwig, Joachim Klimek, Thomas Attin, 5. überarbeitete und erweiterte Auflage, Deutscher Zahnärzteverlag, S. 541). Die Beeinflussung der Arbeitsweise des AirScalers kann zum Beispiel über ein Proportionalventil erfolgen, über das die Krafteinwirkung des Patienten die Pressluftversorgung des AirScalers beeinflusst.

### Dentaleinheit, dentale Behandlungseinheit

Die Dentaleinheit oder dentale Behandlungseinheit besteht meistens aus einem Behandlungsstuhl mit Steuerung und einem Sockel mit gelenkigen Armen, die die Instrumente und Vorrichtungen tragen, die der Zahnarzt zur Behandlung braucht. Das sind unter anderem die Bohrmaschine oder Bohrturbine, die Luft- und Wasserspritze, das Spülbecken, die Instrumentenablage, die Operationsleuchte, ggf. zusätzlich ein Bildschirm und/oder ein Bestrahlungsgerät.

### Kraftmesseinrichtung

Gerät, das geeignet ist, die darauf einwirkende Kraft zu messen, beispielsweise ein Ball oder ein Federelement, das ein Mensch zusammendrücken kann. In dem Ball oder dem Federelement oder daran angeschlossen befinden sich Komponenten (typischerweise elektronisch), die die durch die Kraft hervorgerufene Druckänderung oder Auslenkung messen können und daraus die Kraft ermitteln können.

### Kürette

Küretten werden zur Entfernung subgingivaler Konkremente, nekrotischen, infizierten Wurzelzements und zur Entfernung des Granulationsgewebes und des Taschenepithels verwendet. Sie besitzen eine zierliche Form mit abgerundetem Ende. Es werden Universalküretten und Spezialküretten unterschieden. Universalküretten können aufgrund ihrer Form an allen Quadranten eines Gebisses und dort an allen Zahnflächen eingesetzt werden. Sie sind auf beiden Seiten ihres löffelartigen Arbeitsendes scharf geschliffen. Für besonders schmale oder tiefe Zahnfleischtaschen sind Spezialküretten mit einer kürzeren Arbeitsende oder längerem unteren Schaft geeignet. (Nach: "Einführung in die Zahnerhaltung"; von Elmar Hellwig, Joachim Klimek, Thomas Attin, 5. überarbeitete und erweiterte Auflage, Deutscher Zahnärzteverlag, S. 538)

### Scaler

Ein Scaler ist ein in der Zahnmedizin verwendetes spitzes Handinstrument und dient vor allem der Entfernung supragingivaler Konkremente (oberhalb des Zahnfleischsaumes gelegener Zahnstein). Scaler werden insbesondere zur Entfernung von harten Belägen und zur Grobdepuration verwendet. Sichelscaler besitzen einen dreieckigen Querschnitt, zwei schneidende Kanten und laufen an ihrem Ende spitz zu. Aufgrund ihrer Spitze und Größe ist ein subgingivales Arbeiten ohne Verletzung der Gingiva nicht möglich. Gerade Scaler sind im gesamten Ober- und Unterkieferbereich einsetzbar, gebogene Scaler eignen sich zur Entfernung von Zahnstein im Interdentalbereich. (Nach: "Einführung in die Zahnerhaltung"; von Elmar Hellwig, Joachim Klimek, Thomas Attin, 5. überarbeitete und erweiterte Auflage, Deutscher Zahnärzteverlag, S. 538)

## Patentansprüche

1. Dentales Gerät (200) zur Behandlung eines Patienten, mit einem Mittel (208) zum Steuern des Geräts (200), wobei die Behandlung mit dem Gerät (200) Schmerzen hervorrufen kann; und wobei eine Veränderung einer Arbeitsweise des Geräts (200) geeignet ist, die Schmerzen zu verringern, das Mittel (208) umfassend:
a) eine Kraftmesseinrichtung (204);
b) wobei die Kraftmesseinrichtung (204) durch den Patienten (202) betätigbar ist; und
c) eine Komponente (206), die abhängig von der gemessenen Kraft eine Veränderung der Arbeitsweise des Geräts (200) hervorruft,
d) wobei die Komponente (206) derart ausgebildet ist, dass sie die Arbeitsweise des Geräts (200) graduell verändern kann,
e) Mittel zum Begrenzen des Umfangs, in dem die Komponente (206) abhängig von der gemessenen Kraft die Arbeitsweise des Geräts (200) verändern kann.

2. Dentales Gerät nach Anspruch 1, **gekennzeichnet durch** Mittel (214) zum Verhindern der Veränderung der Arbeitsweise des Geräts (200) durch die Komponente (206).

3. Dentales Gerät nach einem der vorhergehenden Ansprüche, **gekennzeichnet durch** eine Einrichtung zum Aufzeichnen des zeitlichen Verlaufs der Betätigung der Kraftmesseinrichtung (204) durch den Patienten (202).

4. Dentales Gerät nach einem der vorhergehenden Ansprüche, **gekennzeichnet durch** eine Anzeigeeinrichtung, die anzeigen kann, ob die Komponente (206) die Arbeitsweise des Geräts (200) verändert.

5. Dentales Gerät nach einem der vorhergehenden Ansprüche, **dadurch gekennzeichnet, dass** das Gerät (200) ein ultraschallbetriebenes Gerät zur Behandlung einer Parodontitis oder ein AirsScaler ist.

## Claims

1. Dental device (200) for treating a patient with a means (208) for controlling the device (200), wherein the treatment with the device (200) can cause pain, and wherein a change in a mode of operation of the device (200) is suitable for reducing the pain; the means (208) comprising:
a) a force measuring device (204);
b) wherein the force measuring device (204) can be actuated by the patient (202); and
c) a component (206), which effects a change in the operation of the device (200) as a function of the measured force,
d) wherein the component (206) is configured in such a way that it can change the mode of operation of the device (200) gradually.
e) means for limiting the extent to which the component (206) can change the mode of operation of the device (200) as a function of the measured force.

2. Dental device according to Claim 1, **characterized by** means (214) for preventing the change in the mode of operation of the device (200) by the component (206).

3. Dental device according to any of the preceding claims, **characterized by** a device for recording the temporal progression of the actuation of the force measuring device (204) by the patient (202).

4. Dental device according to any of the preceding claims, **characterized by** a display device that can indicate whether the component (206) is changing the mode of operation of the device (200).

5. Dental device according to any of the preceding claims, **characterized in that** the device (200) is an ultrasound-operated device for treating periodontitis or an Air Scaler.

## Revendications

1. Appareil de médecine dentaire (200) pour le traitement d'un patient, comportant un moyen (208) de commande de l'appareil (200), le traitement effectué par l'appareil (200) étant susceptible de provoquer des douleurs ; et une modification d'un fonctionnement de l'appareil (200) étant apte à alléger la douleur, le moyen (208) comprenant :
a) un dispositif de mesure de la force (204),
b) le dispositif de mesure de la force (204) pouvant être actionné par le patient (202) ; et
c) un composant (206) provoquant une modification du fonctionnement de l'appareil (200) en fonction de la force mesurée,
d) le composant (206) étant conçu de manière à pouvoir modifier progressivement le fonctionnement de l'appareil (200),
e) des moyens permettant de limiter l'ampleur avec laquelle le composant (206) peut modifier le fonctionnement de l'appareil (200) en fonction de la force mesurée.

2. Appareil de médecine dentaire selon la revendication 1, **caractérisé par** des moyens (214) visant à empêcher la modification, par le composant (206), du fonctionnement de l'appareil (200).

3. Appareil de médecine dentaire selon une des revendications précédentes, **caractérisé par** un dispositif permettant d'enregistrer dans le temps le cours de l'actionnement du dispositif de mesure de la force (204) effectué par le patient (202).

4. Appareil de médecine dentaire selon une des revendications précédentes, **caractérisé par** un dispositif d'affichage permettant d'afficher si le composant (206) modifie le fonctionnement de l'appareil (200).

5. Appareil de médecine dentaire selon une des revendications précédentes, **caractérisé en ce que** l'appareil (200) est un appareil à ultrasons destiné au traitement de la parodontite ou un détartreur à air.
